(19) **Europäisches Patentamt**

**European Patent Office**

**Office européen des brevets**

(11) **EP 1 598 058 A1**

(12) **DEMANDE DE BREVET EUROPEEN**

(43) Date de publication:
**23.11.2005 Bulletin 2005/47**

(51) Int Cl.⁷: **A61K 7/48**

(21) Numéro de dépôt: **05290620.3**

(22) Date de dépôt: **21.03.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **02.04.2004 FR 0450658**

(71) Demandeur: **L'OREAL**
**75008 Paris (FR)**

(72) Inventeurs:
• **Fagot, Dominique**
  **75020 Paris (FR)**
• **Portes, Pascal**
  **94130 Nogent sur Marne (FR)**

(74) Mandataire: **Leonard, Armelle**
**L'OREAL,**
**River Place,**
**25-29 Quai Aulagnier**
**92600 Asnières-sur-Seine (FR)**

(54) **Utilisation cosmétique d'au moins un inhibiteur de la production et/ou de l'activation des RhoA kinases comme agent destiné à détendre les traits et/ou décontracter la peau**

(57) L'invention concerne l'utilisation dans une composition cosmétique, d'une quantité efficace d'au moins un inhibiteur de la production et/ou de l'activation des RhoA kinases, comme agent destiné à détendre les traits et/ou décontracter la peau.

Ledit composé est notamment destiné à prévenir et/ou lutter contre les signes cutanés du vieillissement, en particulier les rides, et préférentiellement les rides d'expression.

L'invention porte également sur des compositions cosmétiques pour application topique comprenant au moins un inhibiteur de la production et/ou de l'activation des RhoA kinases ainsi que sur un procédé cosmétique de traitement de la peau, notamment de la peau âgée et/ou ridée.

**EP 1 598 058 A1**

**Description**

L'invention concerne l'utilisation dans une composition cosmétique, d'une quantité efficace d'au moins un inhibiteur de la production et/ou de l'activation des RhoA kinases, comme agent destiné à détendre les traits et/ou décontracter la peau.

En particulier ledit inhibiteur est un inhibiteur de l'activation RhoA dépendante des RhoA kinases.

L'invention porte également sur des compositions cosmétiques pour application topique comprenant au moins un inhibiteur de la production et/ou de l'activation des RhoA kinases ainsi que sur un procédé cosmétique de traitement de la peau, notamment de la peau âgée et/ou ridée.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

On sait que les rides ne sont pas toutes les mêmes et que leurs origines sont différentes : certaines sont dues à des modifications morphologiques ou physiologiques en rapport avec l'âge, tandis que d'autres sont dues à des facteurs "aggravants" tels que les mouvements du visage, l'exposition au soleil ou les modifications hormonales.

Les rides faciales varient donc en fonction de l'âge ainsi que du nombre et de l'intensité des facteurs aggravants.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané ou en prévenant sa dégradation.

Bien que ces traitements permettent d'agir sur les rides et ridules dues au vieillissement chronologique ou intrinsèque, ainsi que de celles dues au photo-vieillissement, ils n'ont pas d'effet sur les rides d'expression.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Des liaisons micro-anatomiques entre le derme et le muscle sous-jacent ont été souvent documentées dans les différentes régions du visage. Au niveau de ses liaisons anatomiques les phénomènes continus de contraction et de relaxation produisent des forces de tension dans le tissu conjonctif du derme. Avec le temps, ces forces de traction modifieraient progressivement le phénotype des fibroblastes présents dans la zone de la ride, leur permettant d'acquérir des propriétés contractiles et d'induire un remodelage de la matrice fibreuse dermique. La conséquence serait une formation accrue de lignes d'expression devenant des rides permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, les seuls moyens couramment utilisés pour agir sur les rides d'expression sont, d'une part, la toxine botulique qui est notamment injectée dans les rides de la glabelle (voir J.D. Carruters et al., J. Dermatol. Surg. Oncol., 1992, 18, pp. 17-21) et, d'autre part, des implants dégradables à base de collagène, d'acide hyaluronique ou d'acide polylactique.

En outre, comme alternative à ces techniques médicales nécessitant le recours à un praticien, la Demanderesse a proposé divers composés susceptibles d'offrir un effet dermo-décontractant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'*Iris pallida* (FR-2 746 641).

Il reste toutefois le besoin de disposer de composés efficaces pour prévenir et/ou lutter contre les signes cutanés du vieillissement et lisser ou estomper les rides, en particulier les rides d'expression.

Or la Demanderesse a découvert de façon inattendue que l'inhibition de la voie de signalisation des Rho kinases activées par la protéine RhoA, autrement nommée voie de signalisation des RhoA kinases, permettait de répondre à ce besoin.

En particulier, elle a montré sur un modèle de derme équivalent, que l'utilisation d'un inhibiteur spécifique des RhoA kinases, le (R)-(+)-*trans*-N-(4-Pyridyl)-4-(1-aminoethyl)-cyclohexanecarboxamide dihydrochloride également connu sous le nom de Y-27632, permettait de relaxer et/ou de décontracter les cellules contractiles dermiques supposées être impliquées dans la genèse de la ride d'expression. La Demanderesse a également montré un effet similaire avec un autre inhibiteur des RhoA kinases, le 1(5-isoquinolinesulfonyl)-homopiperazine, également connu sous le nom de HA1077 ou Fasudil. Ces inhibiteurs, qui se lient spécifiquement au niveau du site catalytique des RhoA kinases sont des inhibiteurs de l'activation desdites RhoA kinases.

La décontraction de ces cellules au niveau du derme permettrait ainsi de prévenir et/ou lutter contre les signes du vieillissement cutané, et en particulier de lutter contre les rides d'expression.

Des inhibiteurs de RhoA kinases ont été précédemment décrits dans la littérature comme ayant un effet inhibiteur sur la contraction du muscle lisse suggérant une utilisation comme agent anti-hypertenseur dans la prévention et/ou le traitement thérapeutique des maladies coronariennes, cérébrales et rénales (WO90/05723), le traitement du glaucome (EP1034793, WO2000057914), de l'asthme (JP2000063274), ou de la fibrose pulmonaire (EP1163910).

Mais il n'est nullement suggéré dans l'art antérieur un quelconque effet de ces inhibiteurs de RhoA kinases sur les muscles striés, en particulier sur les muscles peauciers ni sur les cellules contractiles dermiques impliquées dans la génèse de la ride d'expression.

Or, il est connu dans la littérature que les composés efficaces sur le muscle lisse n'ont pas nécessairement d'effet sur le muscle strié, et *vice-versa* (American Journal of Veterinary Research (1996), 57(10), 1497-1500 ; Planta Medica (1970), 18(3), 222-6 ; Nippon Yakurigaku Zasshi (1976), 72(1), 41-52 ; Drug Development Research (1992), 25(2), 161-9).

Ainsi, il n'était pas évident que les inhibiteurs de la production et/ou de l'activation des RhoA kinases selon la présente invention puissent avoir un effet de relaxation sur les muscles striés, en particulier sur les muscles peauciers ou sur les cellules contractiles dermiques impliquées dans la genèse de la ride d'expression, permettant d'envisager leur utilisation comme agent dermo-décontractant dans des compositions cosmétiques à visée anti-âge.

L'invention a donc pour objet l'utilisation dans une composition cosmétique, d'une quantité efficace d'au moins un inhibiteur de la production et/ou de l'activation de RhoA kinases, comme agent destiné à détendre les traits et/ou décontracter la peau.

Par 'inhibiteur de la production et/ou de l'activation des RhoA kinases' selon l'invention, on entend notamment tout agent capable d'inhiber (i) les voies de signalisation conduisant à la transcription ou la traduction des RhoA kinases, (ii) les processus de modification post-traductionelle des RhoAkinases et/ou (iii) l'activation desdites RhoA kinases.

Préférentiellement, ledit inhibiteur est un inhibiteur de l'activation desdites RhoA kinases. Cet inhibiteur de l'activation des RhoA kinases peut être choisi parmi (a) un inhibiteur capable de se lier au site de liaison à l'ATP, empêchant la réaction de phosphorylation desdites kinases, (b) un inhibiteur capable de se lier au site catalytique de ladite kinase induisant un changement conformationnel dans lequel la kinase est inactive, (c) un inhibiteur capable de se lier à des sites de liaison de ses substrats spécifiques, ou (d) un inhibiteur de l'activation par la protéine RhoA desdites RhoA kinases, autrement nommé inhibiteur de l'activation RhoA dépendante desdites RhoA kinases.

Préférentiellement, l'inhibiteur de l'activation desdites RhoA kinases est choisi parmi un agent destiné à se lier au site catalytique desdites RhoA kinases et un agent destiné à inhiber l'activation RhoA dépendante desdites RhoA kinases.

Selon un premier mode de réalisation, on pourra utiliser un inhibiteur capable de se lier au site catalytique des RhoA kinases.

Comme exemples de tels composés, on peut citer les composés trans-4-amino(alkyl)-1-pyridylcarbamoylcyclohexane décrits dans le brevet US 4,997,834 et les composés 4-amino(alkyl)cyclohexane-1-carboxamide décrits dans le brevet US 5,478,838 et/ou leurs dérivés et/ou leurs analogues. On peut également citer le 1(5-isoquinolinesulfonyl)-homopiperazine autrement nommé HA1077 ou Fasudil commercialisé par CALBIOCHEM, et/ou ses dérivés et/ou ses analogues. Plus préférentiellement, le composé sera choisi parmi le (R)-(+)-*trans*-4-(1-aminoethyl)-N-(4-pyridyl)cyclohexanecarboxamide, et/ou ses dérivés et/ou ses analogues.

Par 'dérivés', on entend notamment les sels, les dérivés substitués, les isomères optiques et racémates dudit composé.

Comme sels dudit composé, on peut citer les sels obtenus par addition dudit composé avec un acide inorganique, choisi notamment parmi les acides chlorhydrique, sulfurique, nitrique et phosphorique, ou avec un acide organique, choisi en particulier parmi les acides malonique, succinique, fumarique, lactique, glycolique, citrique et tartrique.

De préférence, il s'agira du (R)-(+)-*trans*-4-(1-aminoethyl)-N-(4-pyridyl)cyclohexanecarboxamide dihydrochloride (Y27632) commercialisé par CALBIOCHEM.

Par 'analogues', on entend notamment les analogues enzymatiques ou biomimétiques dudit composé, capables de se lier au site catalytique des RhoA kinases et d'inhiber ainsi leur activation. De tels analogues peuvent être sélectionnés *in vitro* par des tests de liaison ou d'inactivation des RhoA kinases selon les techniques classiques développées en enzymologie et en biochimie.

Les dérivés et/ou analogues peuvent être d'origine naturelle ou synthétique.

Par 'origine naturelle', on entend un composé à l'état pur ou en solution à différentes concentrations, obtenu par différents procédés d'extraction à partir d'un tissu (peau...) d'origine naturelle.

Par 'origine synthétique', on entend un composé à l'état pur ou en solution à différentes concentrations, obtenu chimiquement ou enzymatiquement ou par production dans un organisme après introduction dans cet organisme des éléments nécessaires à cette production.

Préférentiellement, on utilisera des analogues synthétiques desdits composés.

Selon un autre mode de réalisation, on pourra utiliser un inhibiteur de l'activation RhoA dépendante desdites RhoA kinases.

Cet inhibiteur peut être (a) un agent capable d'inhiber la production et/ou l'activation de la protéine RhoA, ou (b) un inhibiteur capable d'empêcher l'intéraction de la protéine RhoA avec sa cible la RhoA kinase, en se liant par exemple à des sites de liaison spécifiques.

Comme exemple d'inhibiteur de l'activation de la protéine RhoA selon (a), on peut citer un agent capable d'empêcher la transformation de la forme inactive (GDP) de la protéine RhoA en sa forme active liant le GTP, tel qu'un agent à activité ADP-ribosyltransférase conduisant à une ribosylation de la protéine RhoA, dès lors incapable de fixer le GTP.

Comme exemple d'agents à activité ADP-ribosyltransférase, on peut citer l'enzyme C3 exotransférase autrement nommée C3 ou un analogue.

Par analogue de la C3 exotransférase, on entend notamment tout agent ou tout extrait cellulaire ou bactérien non toxique ayant une activité C3 exotransférase. Cette activité C3 exotransférase peut notamment être purifiée à partir des souches de types C et D non toxiques de *Clostridium botulinum* qui la synthétisent. Cette activité C3 est d'ailleurs seulement retrouvée dans ces types C et D, mais n'est pas produite par les souches de types A, B et E, neurotoxiques (Rubin E.J et al., Molecular and cellular biology, Jan 1988, vol.8, n°1, p.418-426).

Les dérivés et/ou analogues des composés Y27632, HA1077 ou de la C3 exotransférase et plus généralement des inhibiteurs de l'activation des RhoA kinases adaptés à la mise en oeuvre de l'invention pourront être :

- préparés par synthèse chimique, enzymatique ou par chimie combinatoire et testés pour leur capacité à se lier et/ou à inactiver respectivement la RhoA kinase ou la protéine RhoA, selon les techniques classiques développées en enzymologie et biochimie ;

    - sélectionnés pour leur activité dermo-décontractante, par exemple sur un derme équivalent.

On utilisera préférentiellement pour évaluer l'activité dermo-décontractante des inhibiteurs selon l'invention, un test comprenant les étapes suivantes :

- on prépare, sur un support, un derme équivalent dans un milieu de culture adéquat comprenant au moins un inhibiteur selon l'invention à tester ;
- on mesure la contraction spontanée des dermes équivalents et on compare la contraction spontanée du derme équivalent préparé avec ledit inhibiteur, par rapport à un derme équivalent préparé sans ledit inhibiteur (derme équivalent témoin) ;
- on sélectionne les composés pour lesquels la contraction spontanée du derme équivalent en présence dudit inhibiteur est diminuée d'au moins 3%, préférentiellement d'au moins 8%, par rapport à la contraction spontanée du derme équivalent témoin.

Par « support », on entend tout support de culture adapté à la préparation du derme équivalent. On peut citer par exemple une plaque de culture de 12 puits (type Costar référence 3512).

Par « derme équivalent », on entend par exemple toute matrice de collagène ensemencée par des cellules de peau, choisies au moins parmi les fibroblastes et les myofibroblastes obtenus par différenciation *in vitro,* sur un support de culture.

On peut citer à titre d'exemple de préparation de derme équivalent les protocoles décrits dans les demandes de brevets (EP-A-285471, EP-A-285474, EP-A-789074, EP-A-502172, EP-A-418035, WO-A-9116010, EP-A-197090, EP-A-20753, FR-A-2665175, FR-A-2689904) ou de préférence le protocole décrit par Asselineau et col., 1987, (Models in dermato., vol.III, Ed. Lowe & Maibach, 1-7).

On peut utiliser alternativement :

- soit un derme équivalent libre : une fois formé, le derme équivalent est immédiatement détaché du support de culture et sa contraction démarre ;
- soit un derme équivalent attaché : une fois formé, le derme équivalent est laissé adhérent au support de culture pendant une certaine durée, puis détaché du support pour que la contraction puisse démarrer.

Par « milieu de culture adéquat », on entend un milieu de culture nutritif qui comprend au moins les éléments nécessaires à la croissance et à la survie des cellules de peau, notamment des fibroblastes ou des myofibroblastes.

On peut citer par exemple comme milieu culture bien connu de l'homme du métier, le milieu MEM (Minimum essential medium).

Les composés inhibiteurs à tester sont utilisés à des concentrations comprise entre $10^{-8}$M et $10^{-3}$M, de préférence entre $10^{-7}$M et $10^{-5}$M.

Par « contraction spontanée » du derme équivalent, on entend l'effet des rétractions intercellulaires établissant des forces de tension entre les cellules et leur milieu environnant. Ces forces induisent une réduction de la surface du derme équivalent au cours du temps, qu'il est possible de mesurer, notamment par analyse d'image. De ces mesures de surface, on déduit un pourcentage de contraction qui permet d'appréhender le phénomène de « micro-tensions » *in vitro.*

La mesure de contraction spontanée des dermes équivalents consiste à mesurer la surface des dermes équivalents et en déduire un taux de contraction spontanée correspondant. Elle peut être réalisée par tout système connu de l'homme du métier.

De préférence, on utilisera un système d'imagerie numérique associé à un logiciel d'analyse d'image. En particulier l'image numérique est acquise au moyen d'une caméra CDD-Iris Sony DXC-107P puis transcrite en mesure de surface et pourcentage de contraction au moyen d'un système d'analyse d'image Zeiss Axiovision 3.0.

Ledit inhibiteur de la production et/ou de l'activation des RhoA kinases selon l'invention est notamment destiné à relaxer et/ou décontracter les cellules contractiles du derme, en particulier les fibroblastes contractiles.

L'invention concerne également l'utilisation dans une composition cosmétique d'au moins un inhibiteur de la production et/ou de l'activation des RhoA kinases, ledit inhibiteur étant destiné à lisser la peau et/ou atténuer ou effacer le microrelief cutané.

Le microrelief cutané selon l'invention est défini par des micro-dépressions à la surface de la peau générées par la contraction des cellules cutanées, en particulier des cellules du derme.

Ledit inhibiteur de la production et/ou de l'activation de RhoA kinases est également destiné à réduire et/ou lisser les rides.

En particulier ledit inhibiteur est destiné à réduire et/ou lisser les rides d'expression.

L'invention concerne également l'utilisation dans une composition cosmétique d'au moins un inhibiteur de la production et/ou de l'activation des RhoA kinases, ladite composition étant adaptée à une administration par voie orale ou à une application par voir topique, préférentiellement à une application par voie topique.

Une formulation adaptée à la voie orale peut être sous forme de dragées, gélules, gels, émulsions, comprimés, capsules ou solutions liquides notamment ampoules buvables, par exemple. En particulier, le(s) actif(s) selon l'invention peuvent être incorporés dans toutes autres formes de compléments alimentaires ou d'aliments enrichis, par exemple des barres alimentaires, ou des poudres compactées ou non.

Un autre objet de l'invention est une composition cosmétique pour application topique comprenant dans un milieu physiologiquement acceptable, une quantité efficace d'au moins un inhibiteur de la production et/ou de l'activation des RhoA kinases tel que défini précédemment.

Par 'milieu physiologiquement acceptable' selon l'invention, on entend un milieu compatible avec la peau et éventuellement avec ses phanères (cils, ongles, cheveux) et/ou les muqueuses.

En particulier, la composition comprend une quantité efficace d'au moins un inhibiteur de l'activation des RhoA kinases choisi parmi le (R)-(+)-*trans*-4-(1-aminoethyl)-N-(4-pyridyl) cyclohexanecarboxamide, le 1 (5-isoquinolinesulfonyl)-homopiperazine, leurs dérivés ou leurs analogues.

La quantité dudit inhibiteur de la production et/ou de l'activation des RhoA kinases est bien entendu fonction de l'effet recherché et peut donc varier dans une large mesure. Pour donner un ordre de grandeur, ledit inhibiteur pourra être dans la composition en une quantité comprise entre 0.0000001% et 10%, préférentiellement entre 0.000001 et 1% du poids total de la composition, encore plus préférentiellement entre 0.00001 et 0.1% du poids total de la composition.

La composition selon l'invention peut se présenter sous toutes les formes galéniques normalement utilisées dans le domaine cosmétique, adaptées à la voie topique.

La composition peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

Elle peut avoir l'aspect d'une crème blanche ou colorée, d'une pommade, d'un lait, d'une lotion, d'un gel, d'un sérum, d'une pâte, d'une mousse, par exemple.

Elle peut également se présenter sous forme solide, en particulier sous forme de stick.

Elle peut encore être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'inhibiteur de l'activation des RhoA kinases.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale ou synthétique (huile de vaseline, isohexadécane), les huiles d'origine végétale (huile d'amande d'abricot, fraction liquide de beurre de karité, d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme conservateurs, on peut citer les esters d'acide para-hydroxybenzoïque, l'octane 1,2-diol, l'iodo-3 propynyl-2 butyl carbamate, le phénoxyéthanol et le gluconate de chlorhexidine.

Comme charges, on peut citer par exemple, les particules de polyamide (Nylon) ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Comme actifs hydrophiles ou lipophiles, il sera avantageux d'introduire dans la composition selon l'invention au moins un composé choisi parmi : d'autres agents dermo-décontractants ; les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents tenseurs ; les agents anti-pollution et/ou anti-radicalaire ; les filtres UV ; et leurs mélanges.
Des exemples de tels composés additionnels sont donnés ci-dessous.

Comme autres agents dermo-décontractants que les inhibiteurs de l'activation des RhoA kinases selon l'invention, on peut citer : les antagonistes des récepteurs associés aux canaux calciques (FR-2 793 681), en particulier le manganèse et ses sels (FR-2 809 005) et l'alvérine et ses sels (FR-2 798 590), notamment le citrate d'alvérine, ; les agonistes des récepteurs associés aux canaux chlore, dont la glycine (EP-0 704 210) et certains extraits *d'Iris pallida* (FR-2 746 641) ; les sapogénines telles que la diosgénine et les extraits naturels en contenant (tels que les extraits de Wild Yam), certaines amines secondaires et tertiaires carbonylées, des sels organiques ou inorganiques de métaux, en particulier le gluconate de manganèse, l'adénosine, ainsi que l'hexapeptide argireline R commercialisé par la société LIPOTEC. On peut également citer l'extrait de *Boswellia serrata* et certaines compositions parfumantes à effet dermo-décontractant.

Par "agent hydratant", on entend :

- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;

- soit un composé activant les glandes sébacées tel que la DHEA, ses dérivés 7-oxydés et/ou 17-alkylés, les sapogénines et la vitamine D et ses dérivés.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Par "agent anti-glycation", on entend un composé prévenant et/ou diminuant la glycation des protéines de la peau, en particulier des protéines du derme telles que le collagène. Des exemples d'agents anti-glycation sont les extraits végétaux de la famille des Ericaceae, tels qu'un extrait de myrtille (*Vaccinium angustifolium*) ; l'ergothionéine et ses dérivés ; et les hydroxystilbènes et leurs dérivés, tels que le resvératrol et le 3,3', 5,5'-tétrahydroxystilbène.

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

Parmi les actifs stimulant les macromolécules du derme ou empêchant leur dégradation, on peut citer ceux qui agissent :

- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9 . On peut citer: les rétinoïdes et dérivés, les oligopeptides et les lipopeptides, les lipoaminoacides, l'extrait de malt commercialisé par la société COLETICA sous la dénomination commerciale Collalift®; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl® ; et les lignanes tels que le sécoisolaricirésinol.

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :

(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

(3) les protéines et hydrolysats de protéines végétales, en particulier de maïs, de seigle, de froment, de sarrasin, de sésame, d'épautre, de pois, de fève, de lentille, de soja et de lupin,

(3) les silicates mixtes, notamment les phyllosilicates et en particulier les Laponites,

(4) les microparticules de cire, choisies par exemple parmi les cires de Carnauba, de Candelila ou d'Alfa,

(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de düsobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

L'invention porte également sur un procédé cosmétique pour réduire les rides et/ou détendre les traits et/ou décontracter la peau, comprenant l'application topique sur la peau d'une composition renfermant, dans un milieu physiologiquement acceptable, une quantité efficace d'au moins un inhibiteur de la production et/ou de l'activation des RhoA kinases tel que défini précédemment.

Ce procédé est adapté au traitement de la peau ridée et/ou âgée et vise notamment à prévenir et/ou réduire les rides d'expression.

En particulier, la composition peut être appliquée sur les zones du visage ou du front marquées par des rides d'expression et/ou sur les personnes présentant des rides d'expression.

Les rides à traiter sont notamment choisies parmi les rides disposées radialement autour de la bouche et/ou des yeux et/ou horizontalement sur le front et/ou situées dans l'espace inter-sourcillier.

La composition pourra être appliquée quotidiennement sur les zones du visage ou du front marquées par des rides d'expression.

L'invention sera maintenant illustrée par les exemples non limitatifs suivants.

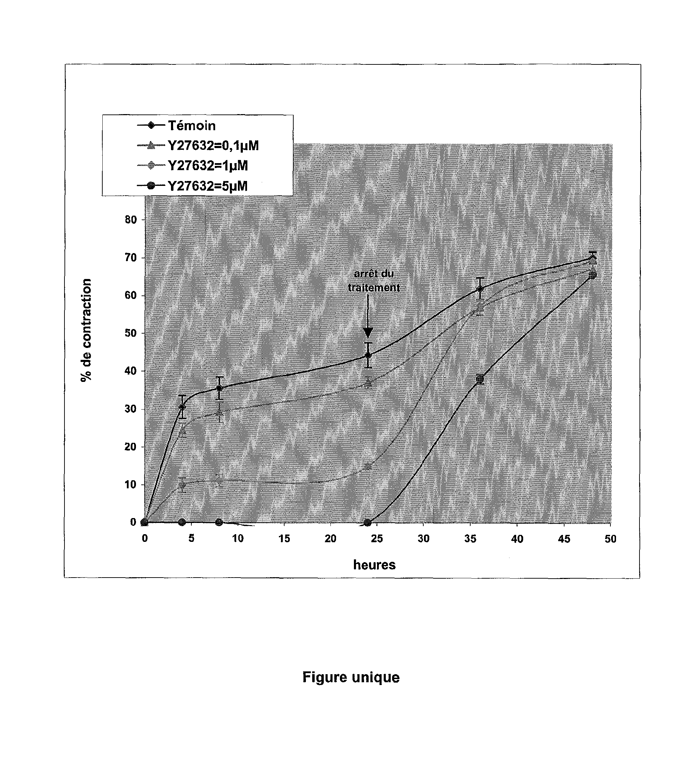

Dans ces exemples, il est fait référence à la Figure annexée qui illustre le taux de contraction au cours du temps d'un derme équivalent traité par le (R)-(+)-*trans*-N-(4-Pyridyl)-4-(1-aminoethyl)-cyclohexanecarboxamide dihydrochloride.

**Exemple 1 : Mise en évidence de l'effet dermo-décontractant du (R)-(+)-*trans*-N-(4-Pyridyl)-4-(1-aminoethyl)-cyclohexanecarboxamide dihydrochloride ou Y27632**

a) Principe du test

Le principe de ce test a consisté à étudier l'effet dermo-décontractant du (R)-(+)-*trans*-N-(4-Pyridyl)-4-(1-aminoethyl)-cyclohexanecarboxamide dihydrochloride (Y-27632) sur un modèle de derme équivalent constitué d'une matrice de collagène ensemencée par des fibroblastes humains normaux.

Ces conditions sont destinées à mimer *in vitro* les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

b) Protocole

Quatre séries de 3 dermes équivalents attachés contenant des fibroblastes humains normaux sont préparés : une série témoin sans aucun traitement, et trois séries traitées par le (R)-(+)-*trans*-N-(4-Pyridyl)-4-(1-aminoethyl)-cydohexanecarboxamide dihydrochloride à différentes concentrations. L'expérience est répétée trois fois.

Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans Y-27632 | 45% |
| Sérum de Veau Foetal: | 9% |
| NaOH (0.1N) : | 5% |
| Acide acétique (1/1000) : | 4% |
| Collagène : | 26% |
| Fibroblastes : | 11 % |

Le derme équivalent traité diffère du derme équivalent témoin en ce que l'on y ajoute des quantités variables de (R)-(+)-*trans*-N-(4-Pyridyl)-4-(1-aminoethyl)-cyclohexanecarboxamide dihydrochloride.

Le collagène utilisé est du collagène de type I (solution commerciale), mais on peut également utiliser du collagène de type III ou IV. Il est extrait de queues de rat ou de peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.

Le protocole est le suivant : dans un tube flacon stérile, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de $1,4 \times 10^5$ cellules pour 1 ml de milieu de culture.

On ajoute alors lentement, contre la paroi du tube de façon à observer l'apparition d'un nuage blanchâtre, un mélange volume/volume de collagène dans de l'acide acétique au 1/1000.

L'ensemble est alors mélangé avec précaution et réparti dans les puits d'une plaque de culture de 12 puits (type Costar référence 3512) à raison de 0.5ml de mélange par $cm^2$. La plaque de culture est alors placée dans un incubateur à 37°C avec 5% de $CO_2$.

Une fois formés après polymérisation du collagène, les dermes équivalents sont laissés adhérents au support de culture pendant 3 jours puis détachés du support pour que la contraction puisse démarrer. Ces dermes équivalents attachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.

L'évaluation de la contraction spontanée des dermes équivalents traité (avec (R)-(+)-*trans*-N-(4-Pyridyl)-4-(1-aminoethyl)-cyclohexanecarboxamide dihydrochloride) et témoin (sans (R)-(+)-*trans*-N-(4-Pyridyl)-4-(1-aminoethyl)-cyclohexanecarboxamide) est réalisée en mesurant leur surface, à différents temps après le début de la contraction spontanée.

Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp-Si)/Sp \times 100$$

où :

'Sp' représente la surface d'un puits de la plaque de culture ; elle correspond à la surface totale du derme équivalent avant contraction ;
'Si' représente la surface du derme équivalent à l'instant i de la cinétique de contraction.

c) Résultats

Comme illustré à la Figure annexée, le taux de contraction du derme équivalent témoin est de 30.5% quatre heures après l'avoir détaché de son support. Il progresse à 35.5% après huit heures pour atteindre 44.2% après vingt-quatre heures.

Dans ce modèle, le (R)-(+)-*trans*-N-(4-Pyridyl)-4-(1-aminoethyl)-cyclohexanecarboxamide dihydrochloride induit une inhibition dose dépendante de la contraction du derme équivalent, qui plus est, réversible.

A la concentration de 0.1µM, le (R)-(+)-*trans*-N-(4-Pyridyl)-4-(1-aminoethyl)-cyclohexanecarboxamide dihydrochloride réduit ce pourcentage de contraction de 6.1 % après quatre heures, 6.4% après huit heures et 7.2% après vingt-quatre heures, par rapport au témoin.

Et à la concentration de 1µM, le (R)-(+)-*trans*-N-(4-Pyridyl)-4-(1-aminoethyl)-cyclohexanecarboxamide dihydrochloride réduit ce pourcentage de contraction de 20.6% après quatre heures, 24.4% après huit heures et 29.3% après vingt-quatre heures, par rapport au témoin.

Ce test démontre ainsi que le (R)-(+)-*trans*-N-(4-Pyridyl)-4-(1-aminoethyl)-cyclohexanecarboxamide entraîne une moindre contraction du derme équivalent, donc un effet relaxant ou dermo-décontractant qui peut être mis à profit dans la préparation de compositions cosmétiques à effet anti-âge, en particulier pour réduire et/ou lisser les rides, et plus particulièrement les rides d'expression.

**Exemple 2: Mise en évidence de l'effet dermo-décontractant du 1(5-isoquinolinesulfonyl)-homopiperazine ou Fasudil (HA1077)**

Le 1(5-isoquinolinesulfonyl)-homopiperazine a été testé à différentes concentrations selon le protocole décrit à l'exemple 1.

Les résultats obtenus sont les suivants :
Le taux de contraction du derme équivalent témoin est de 30.5% quatre heures après l'avoir détaché de son support. Il progresse à 35.5% après huit heures pour atteindre 44.2% après vingt-quatre heures.

Dans ce modèle, le 1(5-isoquinolinesulfonyl)-homopiperazine induit une inhibition dose dépendante de la contraction du derme équivalent.

A la concentration de 0.1µM, le 1(5-isoquinolinesulfonyl)-homopiperazine réduit ce pourcentage de contraction de 2.6% après quatre heures, 2.4% après huit heures et 1.5% après vingt-quatre heures, par rapport au témoin.

A la concentration de 1µM, le 1(5-isoquinolinesulfonyl)-homopiperazine réduit ce pourcentage de contraction de 8% après quatre heures, 9.5% après huit heures et 8.2% après vingt-quatre heures, par rapport au témoin.

A la concentration de 5µM, le 1(5-isoquinolinesulfonyl)-homopiperazine réduit ce pourcentage de contraction de 17% après quatre heures, 19.4% après huit heures et 22.2% après vingt-quatre heures, par rapport au témoin.

Ce test démontre ainsi que le 1(5-isoquinolinesulfonyl)-homopiperazine, bien que moins efficace que le (R)-(+)-*trans*-N-(4-Pyridyl)-4-(1-aminoethyl)-cyclohexanecarboxamide dihydrochloride aux mêmes concentrations, entraîne également une moindre contraction du derme équivalent, donc un effet relaxant ou dermo-décontractant qui peut être mis à profit dans la préparation de compositions cosmétiques à effet anti-âge, en particulier pour réduire et/ou lisser les rides, et plus particulièrement les rides d'expression.

**Exemple 3 : Composition cosmétique**

Cette composition est préparée de manière classique pour l'homme du métier.

| Crème | |
|---|---|
| (R)-(+)-*trans*-N-(4-Pyridyl)-4-(1-aminoethyl)-cyclohexanecarboxamide | 0,001 % |
| Acide stéarique | 3,00 % |

(suite)

| Crème | |
|---|---|
| Mélange de mono-stéarate de glycéryle et stéarate de polyéthylène glycol (100 OE) | 2,50 % |
| Stéarate de polyéthylène glycol (20 OE) | 1,00 % |
| Cyclopentadiméthylsiloxane | 10,00% |
| Charges | 3,00 % |
| Huiles végétales | 7,00 % |
| Huiles synthétiques | 6,00 % |
| Conservateurs | 1,20 % |
| Diméthylsiloxane oxyéthyléné (16 OE) à extrémités méthoxy | 1,00 % |
| Gomme de silicone | 0,20 % |
| Copolymère acrylique en émulsion inverse (Simulgel 600 de SEPPIC) | 1,70 % |
| Alcool stéarylique | 1,00 % |
| Eau | qsp 100 % |

Cette crème est destinée à être appliquée sur le visage et le front pour atténuer les rides d'expression et décontracter le visage.

Des compositions apparentées dans lesquelles le (R)-(+)-*trans*-N-(4-Pyridyl)-4-(1-aminoethyl)-cyclohexanecarboxamide est remplacé par le 1(5-isoquinolinesulfonyl)-homopiperazine peuvent également être préparées.

**Revendications**

1.   Utilisation dans une composition cosmétique d'une quantité efficace d'au moins un inhibiteur de la production et/ou de l'activation des RhoA kinases, comme agent destiné à détendre les traits et/ou décontracter la peau.

2.   Utilisation selon la revendication 1, **caractérisée en ce que** ledit inhibiteur de l'activation des RhoA kinases est un agent destiné à se lier au site catalytique desdites RhoA kinases.

3.   Utilisation selon l'une quelconque des revendications 1 ou 2, **caractérisée en ce que** ledit inhibiteur est choisi parmi le (R)-(+)-*trans*-4-(1-aminoethyl)-N-(4-pyridyl)cyclohexanecarboxamide, le 1 (5-isoquinolinesulfonyl)-homopiperazine et/ou leurs dérivés et/ou leurs analogues.

4.   Utilisation selon la revendication 1, **caractérisée en ce que** ledit inhibiteur de l'activation des RhoA kinases est un agent destiné à inhiber l'activation, par la protéine RhoA, des RhoA kinases.

5.   Utilisation selon la revendication 4, **caractérisée en ce que** ledit agent destiné à inhiber l'activation, par la protéine RhoA, des RhoA kinases est un agent à activité ADP-ribosyltransférase.

6.   Utilisation selon la revendication 5, **caractérisée en ce que** ledit agent à activité ADP-ribosyltransférase est choisi parmi la C3 exotransférase et/ou un analogue.

7.   Utilisation selon l'une quelconque des revendications 1 à 6, **caractérisée en ce que** l'inhibiteur de l'activation des RhoA kinases est susceptible d'être sélectionné par un test comprenant les étapes suivantes :

   -   on prépare, sur un support, un derme équivalent dans un milieu de culture adéquat comprenant au moins un inhibiteur de l'activation des RhoA kinases à tester ;
   -   on mesure la contraction spontanée des dermes équivalents et on compare la contraction spontanée du derme équivalent préparé avec ledit inhibiteur, par rapport à un derme équivalent témoin, préparé sans inhibiteur ;
   -   on sélectionne lesdits inhibiteurs pour lesquels la contraction spontanée du derme équivalent en présence d'inhibiteur est diminuée d'au moins 3%, préférentiellement d'au moins 8%, par rapport à la contraction spontanée du derme équivalent sans inhibiteur.

8.   Utilisation selon l'une quelconque des revendications 1 à 7, **caractérisée en ce que** ledit inhibiteur de la production et/ou de l'activation des RhoA kinases est destiné à lisser la peau et/ou atténuer le microrelief cutané.

**9.** Utilisation selon l'une quelconque des revendications 1 à 7, **caractérisée en ce que** ledit inhibiteur de la production et/ou de l'activation des RhoA kinases est destiné à réduire et/ou lisser les rides.

**10.** Utilisation selon la revendication 9, **caractérisée en ce que** les rides sont les rides d'expression.

**11.** Utilisation selon l'une des revendications 1 à 10, **caractérisée en ce que** ladite composition est adaptée à une administration par voie orale ou à une application topique sur la peau.

**12.** Composition cosmétique pour application topique sur la peau comprenant dans un milieu physiologiquement acceptable, une quantité efficace d'au moins un inhibiteur de l'activation des RhoA kinases choisi parmi le (R)-(+)-*tran*s-4-(1-aminoethyl)-N-(4-pyridyl) cyclohexanecarboxamide, le 1(5-isoquinolinesulfonyl)-homopiperazine , leurs dérivés ou leurs analogues et au moins un adjuvant cosmétique choisi parmi les antioxydants, les parfums, les charges, les filtres, les pigments, les absorbeurs d'odeur et les matières colorantes.

**13.** Composition cosmétique selon la revendication 12, **caractérisée par le fait que** ledit inhibiteur est en une quantité comprise entre 0.0000001 et 10%, préférentiellement entre 0.000001 et 1% en poids par rapport au poids total de la composition, encore plus préférentielement entre 0.00001 et 0.1% du poids total de la composition.

**14.** Composition cosmétique selon l'une des revendications 12 ou 13, **caractérisée par le fait qu'**elle comprend en outre au moins un actif hydrophile ou lipophile choisi parmi d'autres agents dermo-décontractants, les agents hydratants, les agents dépimentants, les agents anti-glycation, les inhibiteurs de NO-synthase, les agents stimulant la synthèse de macromolécules dermiques ou épidermiques et/ou empêchant leur dégradation, les agents stimulant la prolifération des fibroblastes et/ou des kératinocytes ou stimulant la différenciation des kératinocytes, les agents tenseurs, les agents anti-pollution et/ou anti-radicalaire et leurs mélanges.

**15.** Procédé cosmétique pour réduire les rides et/ou détendre les traits et/ou décontracter la peau, comprenant l'application topique sur la peau d'une composition telle que définie dans l'une des revendications 12 à 14.

**16.** Procédé selon la revendication 15, **caractérisé en ce que** les rides sont les rides d'expression.

**17.** Procédé selon l'une quelconque des revendications 15 ou 16, **caractérisé par le fait que** ladite composition est appliquée sur les zones du visage ou du front marquées par des rides d'expression et/ou sur les personnes présentant des rides d'expression.

**Figure unique**

**EP 1 598 058 A1**

**Office européen des brevets**

**RAPPORT DE RECHERCHE EUROPEENNE**

Numéro de la demande

EP 05 29 0620

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.7) |
| A | EP 1 163 910 A (WELFIDE CORPORATION (JP)) 19 décembre 2001 (2001-12-19) * page 16, ligne 49 - ligne 51 * ----- | 1-17 | A61K7/48 |
| A | EP 1 034 793 A (SENJU PHARMA CO (JP); YOSHITOMI PHARMACEUTICAL (JP)) 13 septembre 2000 (2000-09-13) * exemples 1-3,EXPERIMENTALEXAMPLE6 * ----- | 1-17 | |
| A | ANONYMOUS: "Safety Data Sheet from Calbiochem about Y-27632 in solution in water" INTERNET ARTICLE, [Online] XP002305182 Extrait de l'Internet: URL:http://www.emdbiosciences.com/msds/English/688001English.pdf> * le document en entier * ----- | 1-17 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| Munich | 27 septembre 2005 | Diebold, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

15

**ANNEXE AU RAPPORT DE RECHERCHE EUROPEENNE
RELATIF A LA DEMANDE DE BREVET EUROPEEN NO.**

EP 05 29 0620

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-09-2005

| Document brevet cité au rapport de recherche | | Date de publication | Membre(s) de la famille de brevet(s) | | Date de publication |
|---|---|---|---|---|---|
| EP 1163910 | A | 19-12-2001 | AU | 3196900 A | 16-10-2000 |
| | | | CA | 2368675 A1 | 05-10-2000 |
| | | | CN | 1351504 A | 29-05-2002 |
| | | | WO | 0057913 A1 | 05-10-2000 |
| | | | US | 6844354 B1 | 18-01-2005 |
| EP 1034793 | A | 13-09-2000 | AU | 5198199 A | 06-03-2000 |
| | | | CA | 2307285 A1 | 24-02-2000 |
| | | | CN | 1287494 A | 14-03-2001 |
| | | | WO | 0009162 A1 | 24-02-2000 |
| | | | US | 6673812 B1 | 06-01-2004 |

EPO FORM P0460

Pour tout renseignement concernant cette annexe : voir Journal Officiel de l'Office européen des brevets, No.12/82